# EUROPÄISCHE PATENTSCHRIFT

(11) **EP 3 643 270 B1**
(45) Veröffentlichungstag und Bekanntmachung des Hinweises auf die Patenterteilung: **24.11.2021**
(21) Anmeldenummer: 19203145.8
(22) Anmeldetag: 15.10.2019
(51) Int. Cl.: A61C 5/62, A61C 5/64

(54) **VORRICHTUNG MIT EINEM THERMOCHROMEN TEMPERATURINDIKATOR ZUR AUFNAHME, ERWÄRMUNG UND APPLIKATION VON DENTALMATERIALIEN**
DEVICE COMPRISING A THERMOCHROMIC TEMPERATURE INDICATOR FOR RECEIVING, HEATING AND APPLYING DENTAL MATERIALS
DISPOSITIF DOTÉ D'UN INDICATEUR DE TEMPÉRATURE THERMOCHROMIQUE PERMETTANT DE RECEVOIR, CHAUFFER ET APPLIQUER DES MATIÈRES DENTAIRES

(30) Priorität: 22.10.2018 DE 102018126140
(43) Veröffentlichungstag der Anmeldung: 29.04.2020
(73) Patentinhaber: VOCO GmbH, 27472 Cuxhaven (DE)
(72) Erfinder: Leiner, Uwe, 27476 Cuxhaven (DE); Plaumann, Manfred Thomas, 27472 Cuxhaven (DE)

(56) Entgegenhaltungen:
- EP-A1- 3 658 066
- WO-A1-2008/146021
- WO-A1-2015/081109
- CN-U- 207 622 889
- US-A1- 2012 022 541
- US-A1- 2017 056 292

## Beschreibung

Die vorliegende Erfindung betrifft Vorrichtungen mit einem thermochromen Temperaturindikator zur Aufnahme, Erwärmung und Applikation von Dentalmaterialien, ein Verfahren zum Erwärmen von Dentalmaterialien und zum Erkennen der Temperatur der Dentalmaterialien sowie ein entsprechendes Kit.

Als ein Beispiel einer Vorrichtung zur Aufnahme und Applikation eines Dentalmaterials kann eine sogenannte Compule betrachtet werden, aber auch andere Vorrichtungen wie z.B. spritzenförmige Verpackungen oder Kapseln kommen hierfür in Frage.

Compulen sind als Packmittel für Dentalmassen aus dem Stand der Technik hinlänglich bekannt (EP 1 689 314 A1, EP 2 364 123 A1, EP 2 696 794 A1, EP 0 848 935 A1, EP 2 412 448 A2, EP 0 669 113 B1, DE 102 18 859 A1, DE 10 2013 004 077 A1, US 6,877,983 B1). Sie ermöglichen es für jeden Patienten eine vorproportionierte Menge Füllungsmaterial zu lagern, so dass für jeden einzelnen Patienten jeweils ein neues, frisches Material verwendet wird und Querkontamination effektiv ausgeschlossen werden kann. Sie ermöglichen so einen hygienischen Arbeitsablauf.

Um die Dentalmaterialien zu verflüssigen, um sie einfacher ausdrückbar zu machen und ein besseres Anfließverhalten in der Kavität zu ermöglichen, wurden unterschiedliche Lösungen zu ihrer Erwärmung vorgeschlagen.

US 7,097,452 B2 beschreibt eine Compule zum Lagern und Erwärmen von dentalen Kompositen, deren Körper aus einem elektrisch leitenden und einem wärmeleitenden Kunststoff besteht. Zum Erwärmen wird eine externe Stromquelle benötigt.

EP 1 740 119 A1 beschreibt eine Compule zum Lagern und Ausbringen von dentalen Kompositen, deren Körper eine Energieabgabevorrichtung enthält. Diese Energieabgabevorrichtung ist z.B. ein spiralförmig angeordneter Heizdraht zum Erwärmen der Compule und deren Inhalt.

US 7,086,861 B2 offenbart eine Compule zum Lagern und Ausbringen von dentalen Kompositen, deren Körper eine elektrisch betriebene Induktionsspule aufweist, die dazu dient, die Compule und damit ihren Inhalt zu erwärmen.

Neben diesen speziellen, heizbaren Compulen können auch Öfen so ausgestaltet sein, dass sie das Erwärmen der Compulen samt deren Inhalt ermöglichen.

EP 1 151 728 B1 offenbart ein Verfahren zum Erwärmen von Compulen sowie ein entsprechendes Heizgerät.

EP 1 479 356 A2 offenbart ein weiteres Heizgerät zum Erwärmen von Compulen samt deren Inhalt.

Zudem wurden in der noch unveröffentlichten DE 10 2018 114 690 speziell entwickelte Dentalmaterialien beschrieben, die sich durch einen besonders großen Viskositätsabfall bei der Erwärmung auszeichnen. Sie verhalten sich bei hoher Temperatur wie Flow-Komposite und fließen sehr gut an der Zahnsubstanz in der Kavität an. Bei Raumtemperatur verhalten sie sich wie stopfbare Komposite, sind modellierbar und weisen deren sehr gute mechanische Werte auf.

WO 2015/081109 A1 offenbart Spritzen zur Injektion von medizinischen Flüssigkeiten. Diese Spritzen sind aus transparentem Kunststoff oder aus thermochromem Kunststoff, der bei Erwärmung transparent wird, um die enthaltene Flüssigkeit visuell zu identifizieren bzw. zu verifizieren und sie visuell auf Luftblasen oder Verunreinigungen zu prüfen.

Um es dem Anwender möglich zu machen, die Temperatur bzw. die Viskosität eines erwärmten Materials von außen zu erkennen, wurden in WO 2010/003190 A1 Verpackungen mit einem thermochromen Etikett vorgeschlagen.

Nachteilig an diesen thermochromen Etiketten ist, dass sie im Wesentlichen die Temperatur der Oberfläche der Verpackung anzeigen und nicht die Temperatur des Inhalts.

Weiterhin nachteilig ist, dass die auf dem Markt befindlichen Compulen im Hinblick auf ihre äußeren Abmaße sehr engen Toleranzen unterliegen, von denen weder nach oben noch nach unten abgewichen werden kann, ohne dass sich nachteilige Effekte auf die zur Applikation verwendeten Ausbringgeräte ergeben. Dies ist darin begründet, dass die verschiedenen Ausbringgeräte mit verschiedenen Aufnahmemechanismen an verschiedenen Teilen des Compulenkörpers angreifen und da jeweils - je nach Aufnahmemechanismus - bei zu großen Abmaßen oder bei anderem Aufnahmemechanismus bei zu kleinen Abmaßen nicht wie gewollt funktionieren.

Die Verwendung von Etiketten ist in diesem Zusammenhang problematisch. Etikettenmaterialien besitzen eine Dicke im 1/10 mm Bereich. Die Compulen besitzen jedoch nur eine Wandstärke in einer Größenordnung von ca. 1 mm. Bei der Verwendung der Compulen treten hohe Materialspannungen auf, weshalb diese üblicherweise aus hochfesten Kunststoffen hergestellt werden müssen. Wollte man also Etiketten verwenden, so müsste die Wandstärke um die Dicke des Etikettenmaterials reduziert werden, um nicht die äußeren Abmaße zu vergrößern und somit negativ zu beeinträchtigen. Bei einem Etikettenmaterial einer Dicke von 1/10 mm muss die Wandstärke damit also in einer Größenordnung von 10% verringert werden. Damit ist die Verwendung von thermochromen Etiketten auch für die Stabilität des Compulenkörpers nachteilig. Ein nicht ausreichend stabiler Compulenkörper kann während der Anwendung platzen oder reißen. Da das üblicherweise während der Applikation im Mund eines Patienten passieren wird, wäre eine erhöhte Patientengefährdung die Folge.

Da bei der Erwärmung von Dentalmaterialien in Heizöfen ein Temperaturgradient von außen nach innen vorliegt, reagiert ein Etikett auf der Oberfläche sehr viel schneller auf den Temperaturanstieg als das Dentalmaterial im Innern der Verpackung. Es besteht daher die Gefahr, dass der Zahnarzt das Material zu früh aus dem Heizofen entnimmt und das Material zu diesem Zeitpunkt noch nicht seine gewünschte Temperatur erreicht hat. Ist die Temperatur des Materials zu niedrig und damit die Viskosität des Materials noch zu hoch, besteht die Gefahr, dass das Material nicht optimal in der Kavität anfließt und es später an diesen Stellen zu Sekundärkaries kommen kann.

Andererseits tritt sofort nach der Entnahme aus dem Heizofen eine Abkühlung der Compule auf. Auch hier liegt zwischen dem erwärmten Inhalt und der Umgebungstemperatur ein Temperaturgradient vor, auf den ein Etikett auf der Oberfläche der Verpackung schneller reagiert als der Inhalt der Verpackung. Dies kann dazu führen, dass das Etikett dem Zahnarzt fälschlicherweise signalisiert, dass das Material bereits wieder soweit abgekühlt ist, dass es nicht mehr angewendet werden kann, obwohl das Material eigentlich noch die nötige Temperatur aufweist. Dies führt dazu, dass das Material erneut im Heizofen erwärmt wird und sich der Behandlungsablauf so für Patient und Zahnarzt unnötig verzögert.

Es besteht also ein offenkundiger Bedarf an Compulen, die dem Zahnarzt zuverlässig Auskunft geben, ob der Inhalt anwendungsbereit ist oder nicht.

Ein der vorliegenden Erfindung zugrundeliegendes Ziel ist es, eine Vorrichtung zur Aufnahme, Erwärmung und Applikation eines Dentalmaterials und ein Verfahren zur Behandlung eines Dentalmaterials bereitzustellen, die die Nachteile des Standes der Technik vermeiden und dem Zahnarzt die nötige Anwendungssicherheit geben.

Es ist daher gewünscht, eine Lösung vorzustellen, die eine einfache und praxisgerechte Handhabung eines Dentalmaterials im Sinne einer Aufnahme, Erwärmung, Applikation und/oder Behandlung desselben erlaubt.

Erfindungsgemäß wird nach einem ersten Aspekt eine Vorrichtung zur Aufnahme und Applikation von Dentalmaterial vorgeschlagen, wie sie in Anspruch 1 definiert ist, nämlich mit einem Hohlraum zur Aufnahme des Dentalmaterials und einem Kolben, der den Hohlraum an einer Seite verschließt und der entlang der Längsachse in der Vorrichtung beweglich ist, wobei der Kolben zumindest teilweise aus einem thermochromen Material besteht wobei die Vorrichtung im Hohlraum ein strahlungshärtbares Dentalmaterial enthält, welches eine einkomponentige Kompositzusammensetzung ist, umfassend Monomere, Füllstoffe und Initiatoren.

Bevorzugt weist der Kolben unterhalb der Schalttemperatur des thermochromen Materials eine erste Farbe auf und oberhalb der Schalttemperatur des thermochromen Materials eine zweite Farbe auf, die von der ersten Farbe verschieden ist.

Vorzugsweise weist der Kolben in einem ersten Temperaturbereich von 10 bis 30 °C, bevorzugt in einem Temperaturbereich von 15 bis 25 °C, eine erste Farbe auf und in einem zweiten Temperaturbereich von 40 bis 80 °C, bevorzugt in einem Temperaturbereich von 50 bis 70 °C, eine zweite Farbe auf, die von der ersten Farbe verschieden ist.

In einer weiteren bevorzugten Ausführungsform weist der Kolben zudem in einem dritten Temperaturbereich von größer 80 °C eine dritte Farbe auf, die von der zweiten Farbe, bevorzugt von der ersten und der zweiten Farbe, verschieden ist. Ein solcher Kolben erlaubt es dem Zahnarzt nicht nur zu erkennen, wann ein Dentalmaterial ausreichend warm ist, um es verarbeiten zu können, es erlaubt ihm gleichzeitig auch zu erkennen, ob das Dentalmaterial eventuell zu heiß für eine Anwendung am Patienten ist. Es wird dem Zahnarzt also zuverlässig angezeigt, dass er im richtigen Temperaturbereich arbeitet. Bevorzugt weist der Körper der Vorrichtung über einen Temperaturbereich von 10 bis 80°C eine Farbe auf, die der zweiten Farbe des Kolbens im zweiten Temperaturbereich von 40 bis 80 °C, bevorzugt in einem Temperaturbereich von 50 bis 70 °C, entspricht.

Bevorzugt weist der Kolben entlang seiner Längsachse einen Transmissionsgrad von weniger als 10%, vorzugsweise weniger als 5%, besonders bevorzugt von weniger als 1% für Strahlung im Wellenlängenbereich von 250 bis 500 nm, vorzugsweise im Wellenlängenbereich von 450 bis 480 nm, auf.

Bevorzugt umfasst eine erfindungsgemäße Vorrichtung eine wasserdampf- und/oder lichtundurchlässige Verpackung, d.h. sie ist wasserdampf- und/oder lichtundurchlässig verpackt. Solche wasserdampf- und/oder lichtundurchlässigen Verpackungen sind vorzugsweise Blister oder Folienbeutel.

Bevorzugt ist eine erfindungsgemäße Vorrichtung ausgewählt aus der Gruppe bestehend aus Spritzen, Applikationskanülen, Kapseln und Compulen.

Obwohl nur oberflächennahe thermochrome Pigmente für den Anwender sichtbar sind, hat sich erstaunlicherweise gezeigt, dass die Korrelation der angezeigten Temperatur mit der tatsächlichen Temperatur des Compuleninhalts, mit einem thermochrom durchgefärbten Kolben genauer zu erzielen ist, als dies mit separaten Bauteilen, z.B. Etiketten, der Fall ist. Zusätzlich erfolgt das Ansprechverhalten bei einem thermochrom durchgefärbten Kolben auf direkterem Weg.

Es ist anzunehmen, dass der Wärmeübergang innerhalb nur eines Material (dem Kolbenmaterial) im Vergleich zum Wärmeübergang von einem Material über eine Klebstoffschicht auf das andere Material (dem Etikettenmaterial) zu diesem unterschiedlichen Ansprechverhalten führt.

In einer bevorzugten Variante der obigen Ausgestaltung besteht der Kolben aus einem mit einem thermochromen Material eingefärbten Kunststoff. Bevorzugt ist das thermochrome Material ausgewählt aus der Gruppe bestehend aus anorganischen Pigmenten umfassend Metallsalze oder Metalloxide, bei denen durch einen Phasenübergang, eine Änderung der Ligandengeometrie, eine Änderung der Koordinationszahl und/oder eine Änderung des Kristallfeldes ein Farbübergang stattfindet, organischen Pigmenten umfassend thermochrome Flüssigkristalle, konjugierte Polymere und Leukofarbstoffe sowie Kombinationen davon.

Als thermochrome Farbstoffe im Sinne eines Beispiels eines thermochromen Materials, das im Rahmen der vorliegenden Erfindung genutzt werden kann, können anorganische oder organische Pigmente eingesetzt werden.

Anorganische Pigmente sind Metallsalze oder Metalloxide, bei denen durch Phasenübergänge, Änderung der Ligandengeometrie, Änderung Koordinationszahl oder Änderung des Kristallfeldes ein Farbübergang stattfindet.

Organische Pigmente umfassen thermochrome Flüssigkristalle, konjugierte Polymere und Leukofarbstoffe. Thermochrome Flüssigkristalle sind beispielsweise Cholesterinderivate oder Cyanobiphenyle. Konjugierte Polymere können durch Konformationsänderungen Farbänderungen aufweisen. Als Leukofarbstoffe können Systeme auf Basis von Spiropyranen (Formel 1), Spirooxazinen (Formel 2), Salicyl-Schiff'schen Basen (Formel 3), Bianthronen (Formel 4), Indolylphthaliden (Formel 5) oder Fluoranen (Formel 6) eingesetzt werden.

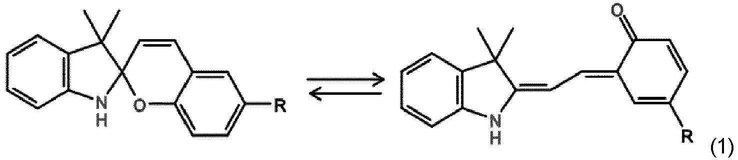

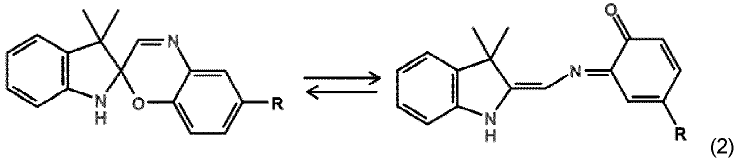

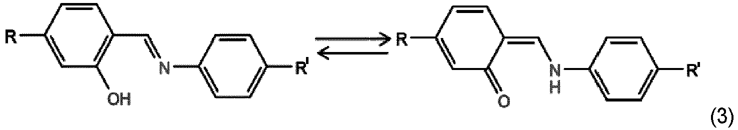

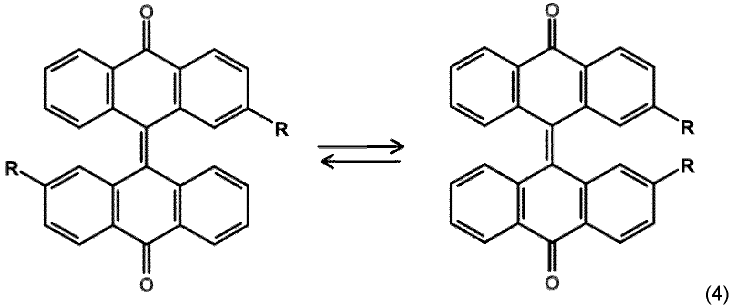

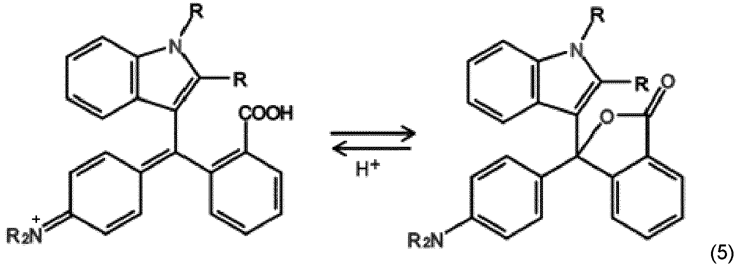

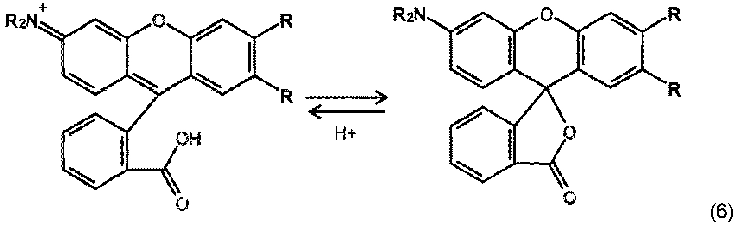

Solche Systeme bestehen in der Regel aus einem Elektronendonor (Spiropyrane, Spirooxazine, Salicyl-Schiff'sche Basen, Bianthrone, Indolylphthalide oder Fluorane) und einem Elektronenakzeptor. Bei den Elektronendonoren lassen sich durch geeignete Wahl der Substituenten unterschiedliche Farben einstellen. Zusätzlich zu den in den Formeln vereinfacht dargestellten Positionen für die Substituenten sind auch andere Positionen denkbar. Als Elektronenakzeptoren werden Phenole, Azole oder organische Säuren eingesetzt. Beispielhafte Phenole sind Phenylphenol, Bisphenol A, Bisphenol AP, Bisphenol AF, Bisphenol FL, Cresol, Resorcinol, Phloroglucinol, Phenol, Phenololigomere, Naphthol, 1,5-Dihydroxynaphthalin, Pyrocatechol und Pyrogallol. Beispielhafte Azole sind Benzotriazole, wie 5-Chlorbenzotriazol, 4-Laurylaminosulfobenzotriazol, 5-Butylbenzotriazol, Dibenzotriazol, 2-Oxybenzotriazol,5-Ethoxycarbonylbenzotriazol,5,5'-Methylenbisbenzotriazol, Imidazole, wie Oxybenzimidazol, und Tetrazole. Die organischen Säuren umfassen beispielsweise aromatische und aliphatische Carbonsäuren und substituierte Derivate davon. Beispiele für aromatische Carbonsäuren sind Salicylsäure, Methylenbissalicylsäure, Resorcylsäure, Gallussäure, Benzoesäure, p-Oxybenzoesäure, Pyromellitsäure, Naphthoesäure, Gerbsäure, Toluylsäure, Trimellithsäure, Phthalsäure, Terephthalsäure und Anthranilsäure. Beispiele für aliphatische Carbonsäuren sind Säuren mit 1 bis 20 Kohlenstoffatomen, vorzugsweise 3 bis 15 Kohlenstoffatomen, wie beispielsweise Stearinsäure, 1,2-Hydroxystearinsäure, Weinsäure, Zitronensäure, Oxalsäure und Laurinsäure.

Das thermochrome Material bzw. die thermochromen Materialien können als solche oder in mikroverkapselter Form eingesetzt werden. Vorteilhafterweise wird aus dem thermochromen Material oder dem mikroverkapselten thermochromen Material zunächst ein Masterbatch bzw. ein Farbgranulat hergestellt, welches dann dem einzufärbenden Kunststoff zugegeben wird.

Geeignete Kunststoffe für den Compulenkörper bzw. die Wandung sowie für den Kolben einer erfindungsgemäßen Vorrichtung sind die Standardthermoplaste Polyethylen (PE), Polypropylen (PP), Polystyrol (PS), Polyvinylchlorid (PVC) sowie Acrylnitril-Butadien-Styrol-Copolymer (ABS). Weiter geeignet sind die technischen Thermoplaste Polycarbonat (PC), Styrol-Acrylnitril-Copolymer (SAN), Polymethylmethacrylat (PMMA), Polyamid (PA), Polyoxymethylen (POM), Polybutylenterephthalat (PBT), Polypropylenterephthalat (PPT), Polyethylenterephthalat (PET) sowie Polyphthalamid (PPA). Geeignet sind auch die Hochleistungsthermoplaste Polyetherketon (PEK), Poly(etheretherketon) (PEEK), Poly(etherketonketon) (PEKK), Poly(etheretheretherketon) (PEEEK), Poly(etheretherketonketon) (PEEKK), Poly(etherketon-etherketonketon) (PEKEKK) sowie Polyaryletherketone (PAEK). Außerdem können auch faserverstärkte Kunststoffe verwendet werden.

Bevorzugt besteht der Körper bzw. die Wandung einer erfindungsgemäßen Vorrichtung aus Polyamid (PA) oder Polybutylenterephthalat (PBT).

Bevorzugt besteht der Kolben einer erfindungsgemäßen Vorrichtung aus Polyethylen (PE), Polyproplylen (PP), Polyvinylchlorid (PVC), Polystyrol (PS), Polyterafluoroethylen (PTFE), Thermoplastischen Elastomeren (TPE), Liquid Silicone Rubber (LSR), Silikon, Ethylen-Propylen-Dien-Kautschuk (EPDM), Nitril-Butadien-Kautschuk (NBR) oder Fluorkautschuk (FKM), vorzugsweise aus PE, PP oder PVC.

Es hat sich gezeigt, dass bei Verwendung eines thermochromen Kolbens außerdem der Vorteil besteht, dass dieser die Temperatur des Dentalmaterials im Kapselinneren, das physischen Kontakt zum Kolben hat, sehr präzise anzeigen kann. Dabei ist die Anzeige umso präziser bzw. zeitgenauer, je besser die Wärmeleitfähigkeit des Kolbenmaterials ist.

In einer bevorzugten Ausführung kann der Kolben aus verschiedenen Materialkombinationen bestehen. Insbesondere kann die nach außen gerichtete Fläche des montierten Kolbens aus einer Schicht eines thermochromen Werkstoffes bestehen, wohingegen der Rest des Kolbens aus einem nicht thermochromen Werkstoff besteht, der eine bessere Abdichtwirkung, Lichtdichtigkeit gegen bestimmte Wellenlängen oder eine bessere Wärmeleitfähigkeit als das thermochrome Material besitzt. Die verschiedenen Materialien lassen sich beispielsweise im 2-Komponenten-Spritzgußverfahren stoffschlüssig als ein untrennbares Bauteil herstellen.

Bevorzugt wird der Kolben einer erfindungsgemäßen Vorrichtung im 2-Komponenten-Spritzgussverfahren aus zwei verschiedenen Materialien hergestellt wird, von denen das der Außenseite zugewandte Material thermochrome Eigenschaften hat.

Das der Innenseite zugewandte Material kann beispielsweise auch mit verbesserten Wärmeleiteigenschaften ausgewählt werden, um das Ansprechverhalten noch weiter zu verbessern.

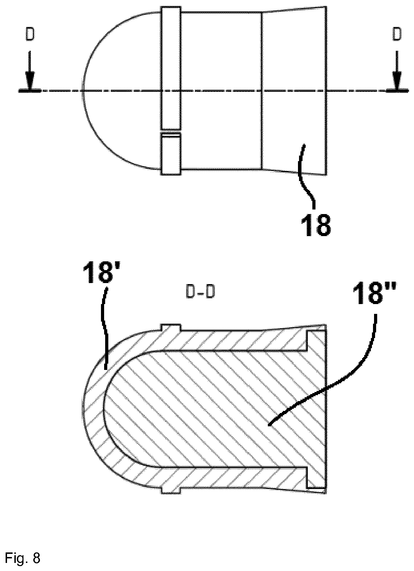

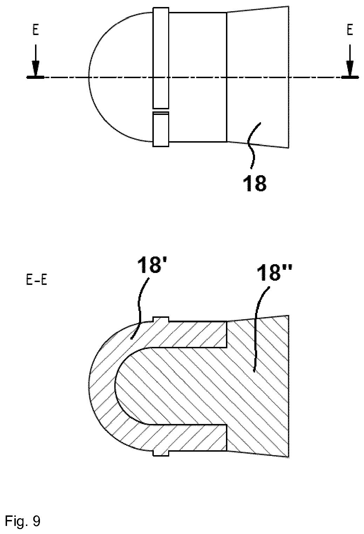

In den Figuren 8 und 9 ist schematisch ein solcher im 2-Komponenten-Spritzgussverfahren hergestellter Kolben 18 dargestellt. Das eine Material 18' ist dem Innenraum und dem darin befindlichen Dentalmaterial zugewandt. Das zweite, thermochrome Material 18" ist der Außenseite zugewandt und dem Anwender sichtbar.

Eine erfindungsgemäße Vorrichtung enthält im Hohlraum ein strahlungshärtbares Dentalmaterial, welches eine einkomponentige Kompositzusammensetzung ist, umfassend (A) Monomere, (B) Füllstoffe und (C) Initiatoren.

Die dentale, lichthärtbare, einkomponentige Kompositzusammensetzung vorzugsweise zur Herstellung eines dentalen Füllungsmaterials, Unterfüllungsmaterials, Befestigungsmaterials oder Fissurenversieglers, kann hierbei beispielsweise umfassen:
(A) Monomere in einer Menge von 6 bis 35 Gew.-%, bezogen auf die Kompositzusammensetzung, vorzugsweise 10 bis 35 Gew.-%, besonders bevorzugt 10 bis 25 Gew.-%,
(B) Füllstoffe in einer Menge von 65 bis 93 Gew.-%, vorzugsweise 65 bis 89 Gew.-%, besonders bevorzugt 75 bis 89 Gew.-%, bezogen auf die Kompositzusammensetzung,
(C) Initiatoren in einer Menge von 0,001 bis 3 Gew.-%, bezogen auf die Menge der Kompositzusammensetzung,
(D) weitere Additive in einer Menge von 0,001 bis 5 Gew.-%, bezogen auf die Menge der Kompositzusammensetzung.

In einer besonderen Variante umfasst Bestandteil (A) der dentalen, lichthärtbaren, einkomponentigen Kompositzusammensetzung die Mischung von zumindest (A-i) einer ersten Monomerensubstanz und (A-ii) einer zweiten Monomerensubstanz, wobei die Viskosität der zweiten Monomerensubstanz (A-ii) bei 20 °C größer ist als 100 Pa·s, die Viskosität der ersten Monomerensubstanz (A-i) bei 20 °C größer ist als 100 mPa·s, die Viskosität der zweiten Monomerensubstanz (A-ii) bei 20 °C größer ist als die der ersten Monomerensubstanz (A-i) und das Massenverhältnis der ersten Monomerensubstanz (A-i) zur zweiten Monomerensubstanz (A-ii) im Bereich von 2:1 bis 1:10 liegt, wobei die zweite Monomerensubstanz (A-ii) vorzugsweise zumindest 40 Gew.-% 2,2-Bis[4-(2-hydroxy-3-methacryloyloxypropoxy)phenyl]propan (Bis-GMA) und/oder lichthärtbare Derivate des Diisocyanatodiphenylmethans (MDI) und/oder lichthärtbare Derivate des Tetramethyl-m-xylylendiisocyanats (TMXDI) enthält, wobei die Gew.-%-Angabe auf die Gesamtmasse der Monomere (A) bezogen ist.

Die Untersuchungen, welche die Erfinder im Rahmen der vorliegenden Erfindung durchgeführt haben, zeigen, dass bei Einsatz der Bestandteile (A) bis (D) in den angegebenen Mengen (vorzugsweise in den als bevorzugt angegebenen Mengenbereichen) besonders effizient vorgesehenen Viskositätsmaßgaben eingestellt werden können.

In einer bevorzugten Ausgestaltung bestehen die Monomere (A) aus
(A1) 10 bis 60 Gew.-%, vorzugsweise 20 bis 50 Gew.-%, besonders bevorzugt 25 bis 40 Gew.-%, lichthärtbare bi- oder tricyclische Verbindungen Q(YₓZₑ)_{b}, wobei gilt
   Q bedeutet ein gesättigtes oder olefinisch ungesättigtes bi- oder tricyclisches Strukturelement, jeder Index b ist eine natürliche Zahl, ausgewählt aus der Gruppe der natürlichen Zahlen 1, 2, und 3, jedes Z bedeutet eine lichthärtbare Gruppe, jeder Index e ist eine natürliche Zahl, ausgewählt aus der Gruppe der natürlichen Zahlen 1, 2 und 3, jedes Y bedeutet in der Struktur Q(YₓZₑ)_{b} bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und jeder Index x ist 0 oder 1,
(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl] propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),
(A3) 0 bis 15 Gew.-%, vorzugsweise 0 bis 10 Gew.-%, besonders bevorzugt 0 bis 5 Gew.-% weitere radikalisch polymerisierbare Monomere, die nicht (A1) oder (A2) zuzuordnen sind,
wobei die Gew.-%-Angaben von (A1), (A2) und (A3) auf die Gesamtmasse der Monomere (A) bezogen sind.

Die dentale, lichthärtbare, einkomponentige Kompositzusammensetzung kann ferner beispielsweise umfassen (A) Monomere, (B) Füllstoffe und, (C) Initiatoren, wobei die Monomere (A) bestehen aus
(A1) 10 bis 60 Gew.-%, vorzugsweise 20 bis 50 Gew.-%, besonders bevorzugt 25 bis 40 Gew.-%, lichthärtbare bi- oder tricyclische Verbindungen Q(YₓZₑ)_{b}, wobei gilt, Q bedeutet ein gesättigtes oder olefinisch ungesättigtes bi- oder tricyclisches Strukturelement,jeder Index b ist eine natürliche Zahl, ausgewählt aus der Gruppe der natürlichen Zahlen 1, 2, und 3, jedes Z bedeutet eine lichthärtbare Gruppe, jeder Index e ist eine natürliche Zahl, ausgewählt aus der Gruppe der natürlichen Zahlen 1, 2 und 3, jedes Y bedeutet in der Struktur Q(YₓZₑ)_{b} bei x = 1 ein Strukturelement, das das Strukturelement Q mit e Strukturelementen Z verbindet und das eine gerade oder verzweigte Alkylengruppe bedeutet, wobei die Alkylengruppe durch Sauerstoffatome unterbrochen sein kann und jeder Index x ist 0 oder 1,
(A2) 40 bis 90 Gew.-%, vorzugsweise 50 bis 80 Gew.-%, besonders bevorzugt 60 bis 75 Gew.-%, 2,2-Bis[4-(2-hydroxy-3-methacryloyloxypropoxy)phenyl] propan (Bis-GMA) und/oder lichthärtbare Derivate des Diisocyanatodiphenylmethans (MDI) und/oder lichthärtbare Derivate des Tetramethyl-m-xylylendiisocyanats (TMXDI),
(A3) 0 bis 15 Gew.-%, vorzugsweise 0 bis 10 Gew.-%, besonders bevorzugt 0 bis 5 Gew.-%, ganz besonders bevorzugt 0 Gew.-% weitere radikalisch polymerisierbare Monomere, die nicht (A1) oder (A2) zuzuordnen sind,
wobei die Gew.-%-Angaben von (A1), (A2) und (A3) auf die Gesamtmasse der Monomere (A) bezogen sind.
- Fig. 1: eine schematische Darstellung zur Illustration eines ersten Ausführungsbeispiels der erfindungsgemäßen Vorrichtung,
- Fig. 2: eine weitere schematische Darstellung zur Illustration eines ersten Ausführungsbeispiels der erfindungsgemäßen Vorrichtung,
- Fig. 3: eine schematische Darstellung einer erfindungsgemäßen Vorrichtung in einer Heizeinheit,
- Fig. 4: eine weitere schematische Darstellung einer erfindungsgemäßen Vorrichtung in einer Heizeinheit,
- Fig. 5: eine weitere schematische Darstellung einer erfindungsgemäßen Vorrichtung in einer Heizeinheit,
- Fig. 6: eine weitere schematische Darstellung einer erfindungsgemäßen Vorrichtung in einer Heizeinheit,
- Fig. 7: Temperaturverlauf von Dentalmaterial, Kolben und Etikett während Aufheiz- und Abkühlphase,
- Fig. 8: eine schematische Darstellung eines 2-Komponenten-Spritzguss-Kolbens,
- Fig. 9: eine weitere schematische Darstellung eines 2-Komponenten-Spritzguss-Kolbens.

In den beiliegenden Zeichnungen sowie den Erläuterungen zu diesen Zeichnungen sind einander entsprechende bzw. in Beziehung stehende Elemente - soweit zweckdienlich - mit jeweils entsprechenden oder ähnlichen Bezugszeichen gekennzeichnet, auch wenn sie in unterschiedlichen Ausführungsbeispielen zu finden sind.

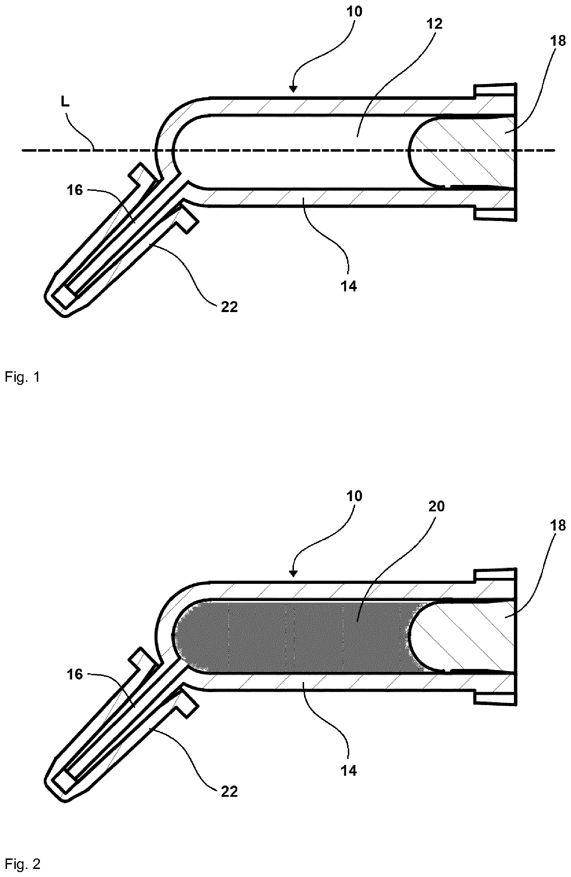

Fig. 1 zeigt eine schematische Darstellung zur Illustration eines ersten Ausführungsbeispiels der erfindungsgemäßen Vorrichtung.

Im ersten Ausführungsbeispiel ist die Vorrichtung 10 als Compule ausgeführt, die einen Hohlraum 12 aufweist, der zur Aufnahme von Dentalmaterial (in Fig. 1 nicht gezeigt) vorgesehen ist. Der Hohlraum 12 wird von einem Körper 14 der Compule 10 umschlossen, der wiederum mit einer Auslassöffnung 16 versehen ist, die mit einer Kappe 22 verschließbar ist. Der zur Verfügung stehende Teil des Hohlraums 12 wird zudem von einem Kolben 18 begrenzt, der innerhalb des Körpers 14 der Compule 10 angeordnet ist, um entlang einer Längsachse L der Compule 10 beweglich zu sein, so dass im Hohlraum 12 befindliches Dentalmaterial durch die Auslassöffnung 16 ausgebracht werden kann.

Da Compulen als solche, ihr Aufbau und ihre Verwendung bekannt sind, kann hier auf eine weitere Erläuterung verzichtet werden.

Es sei allerdings darauf hingewiesen, dass die Beschreibung der Erfindung anhand von Ausführungsbeispielen in Form einer Compule nicht als Beschränkung zu verstehen ist, da die Erfindung allgemein eine zur Aufnahme und Applikation von Dentalmaterial geeignete Vorrichtung vorsieht, die nicht nur durch eine Compule realisiert werden kann, sondern beispielsweise auch durch eine Spritze, Kapsel oder eine Applikationskanüle.

Fig. 2 zeigt eine weitere schematische Darstellung zur Illustration eines ersten Ausführungsbeispiels der erfindungsgemäßen Vorrichtung und deren Verwendung. Im Hohlraum 12 ist hier das Dentalmaterial 20 dargestellt.

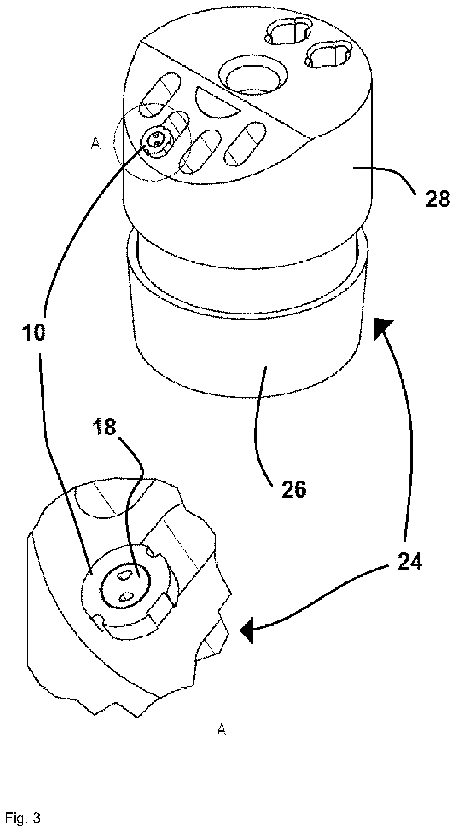

Fig. 3 zeigt eine räumliche Darstellung der erfindungsgemäßen Vorrichtung 10 in einem Heizofen 24. Der Heizofen 24 besteht aus einer Basis 26, die Heizelemente sowie Mess- und Regeltechnik enthält. Auf der Basis 26 steht ein Aufnahmeblock 28 mit verschiedenen Kavitäten, in die verschiedenartige erfindungsgemäße Vorrichtungen platziert werden können.

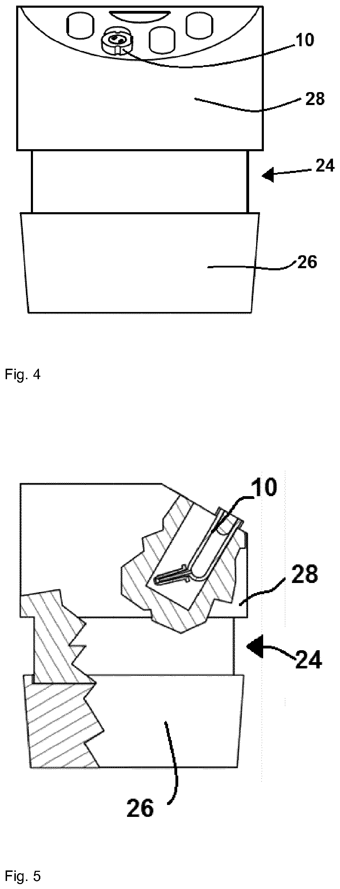

Die Figuren 4 bis 6 zeigen entsprechende Darstellungen der erfindungsgemäßen Vorrichtung 10 in einem Heizofen 24 aus unterschiedlichen Perspektiven.

In diesem Beispiel befindet sich eine Compule 10 mit einem thermochromen Kolben 18 in einer der Aufnahmen. Die Basis 26 wird eingeschaltet und überträgt Wärmeenergie an den darauf stehenden Aufnahmeblock 28. Dieser ist zweckmäßigerweise aus einem gut wärmeleitfähigen Material, z.B. einem Metall hergestellt. Dieser Aufnahmeblock 28 wird somit auf eine voreingestellte Temperatur aufgeheizt. Sobald diese erreicht ist, hält die Regelungstechnik die Temperatur konstant.

Nach dem Aufheizen auf z.B. 60°C platziert der Anwender die erfindungsgemäße Vorrichtung 10 in der Aufnahme. Der nach außen zeigende Teil des thermochromen Kolbens (18, 18") ist in diesem Fall gut sichtbar. Die erfindungsgemäße Vorrichtung wird nun durch den Aufnahmeblock durch den physischen Kontakt, aber auch durch Konvektions- und Strahlungswärme erhitzt. Die Wärmeenergie dringt über die Wandung bzw. den Körper 14 der erfindungsgemäßen Vorrichtung zum Dentalmaterial 20, das sich im Innenraum 12 der Vorrichtung befindet, ebenso aber auch zum thermochromen Kolben 18. Dabei erwärmen sich Kolben und Material gleichermaßen. Der Kolben ändert am Umschlagpunkt aufgrund seiner thermochromen Eigenschaften seine Farbe.

Dieser Umschaltpunkt ist so gewählt, dass er eine gewünschte Mindestverarbeitungstemperatur anzeigt für den Fall, dass das Material über einen großen Verarbeitungstemperaturbereich verfügt.

Für Materialien, die über einen schmalen Verarbeitungstemperaturbereich verfügen, ist der Umschaltpunkt so gewählt, dass er an der gewünschten Verarbeitungstemperatur umschlägt.

Zur besseren Kontrolle kann der Heizblock 24 eine oder mehrere Farbmarkierungen enthalten, anhand derer der Anwender den vollständigen Farbumschlag mit dem thermochromen Kolben vergleichen kann. Diese Farbmarkierung kann beispielsweise auch auswechselbar gestaltet sein.

Darüber hinaus ist es möglich, den Compulenkörper derart einzufärben, dass Farbe des Compulenkörpers der Farbe des umgeschlagenen thermochromen Kolbens entspricht. Dadurch kann auf die Verwendung der zuvor beschriebenen separaten Farbmarkierung verzichtet werden.

Bevorzugt umfasst ein erfindungsgemäßes Kit
- eine erfindungsgemäße Vorrichtung,
- ein Dentalmaterial, enthalten in der Vorrichtung und
- eine Heizeinheit.

Bevorzugt weist die Heizeinheit eines erfindungsgemäßen Kits ein Referenzetikett, eine Referenzmarkierung oder eine Referenzskala auf, die der Farbe des Kolbens eine Temperatur zuordnet.

Erfindungsgemäße Vorrichtungen können darüber hinaus vorteilhaft in Verfahren zum Erwärmen von Dentalmaterialien eigesetzt werden.

Ein solches Verfahren zum Erwärmen von Dentalmaterialien umfasst die folgenden Schritte
a) Bereitstellen einer erfindungsgemäßen Vorrichtung, enthaltend ein Dentalmaterial,
b) Bereitstellen einer Heizeinheit,
c) Platzieren der Vorrichtung enthaltend das Dentalmaterial in der Heizeinheit,
d) Erwärmen der Vorrichtung und des darin enthaltenen Dentalmaterials von einer ersten Temperatur unterhalb der Schalttemperatur des thermochromen Kolbenmaterials auf eine zweite Temperatur oberhalb der Schalttemperatur des thermochromen Kolbenmaterials,
e) Visuelles Erkennen, dass eine Temperatur oberhalb der Schalttemperatur erreicht ist am Farbwechsel des thermochromen Kolbenmaterials.

In einem bevorzugten erfindungsgemäßen Verfahren wird in Schritt d) von einer ersten Temperatur im Bereich von 10 bis 30 °C, vorzugsweise im Bereich von 15 bis 25 °C, auf eine zweite Temperatur im Bereich von 40 bis 80 °C, vorzugsweise im Bereich von 50 bis 70 °C, erwärmt.

In einem bevorzugten erfindungsgemäßen Verfahren wird in Schritt e) die Farbe des thermochromen Kolbenmaterials mit der Farbe einer, bevorzugt auf der Heizeinheit angebrachten, Referenzmarkierung oder Referenzskala abgeglichen, die die Farbe in Bezug zur Kolbentemperatur setzt.

In einem bevorzugten erfindungsgemäßen Verfahren wird in Schritt e) visuell anhand der Farbe des thermochromen Kolbenmaterials erkannt, dass der zweite Temperaturbereich von 40 bis 80 °C weder unter- noch überschritten wird.

### Bezugszeichenliste

- 10: Compule
- 12: Hohlraum
- 14: Körper
- 16: Auslassöffnung
- 18: Kolben
- 18': erstes Kolbenmaterial
- 18": zweites, thermochromes Kolbenmaterial
- 20: Dentalmaterial
- 22: Kappe
- 24: Heizeinheit
- 26: Basis
- 28: Aufnahmeblock
- L: Längsachse

### Ausführungsbeispiele

Die Ausführungsbeispiele wurden am Dentalkomposit Grandio SO (VOCO GmbH) durchgeführt. Das Compulenmaterial besteht aus Polyamid (PA). Das Dentalkomposit ist Grandio SO in der Farbe A2 (Charge 1822301). Als Heizeinheit wurde der Caps Warmer (VOCO GmbH) verwendet. Die Heizeinheit wurde für 30 Minuten auf 68 °C vorgeheizt.

### Beispiel 1

Aus einer Compule Grandio SO (VOCO GmbH) wurde der Kolben entfernt. Durch diese hintere Öffnung wurde ein Thermoelement (DTH-TYPK-4K mit Messfühler NiCrNi Typ K) im Dentalmaterial platziert. Die Compule wurde in der auf 68 °C vorgeheizten Heizeinheit (Caps Warmer - VOCO GmbH) platziert und gleichzeitig wurde die Temperaturmessung gestartet. Nach drei Minuten wurde die Compule aus der Heizeinheit entfernt und der Temperaturverlauf weiter aufgezeichnet.

### Beispiel 2

Aus 5 Gew.-% thermochromen Masterbatch und 95 Gew.-% Polyethylen wurden Folien mit einer Dicke von 100 µm hergestellt. Daraus wurden Etiketten mit einer Größe von 10 mm x 10 mm geschnitten und auf der Seitenwand einer Grandio SO Compulen angebracht. Die Compule wurde in der auf 68 °C vorgeheizten Heizeinheit (Caps Warmer - VOCO GmbH) platziert. Alle 10 Sekunden wurde die Temperatur des Etiketts gemessen (Optris CT LT22 CF). Zusätzlich wurde die Farbe des Etiketts beobachtet. In einem Temperaturbereich von 45 bis 50 °C erfolgte ein Farbumschlag von violett nach rot. Nach drei Minuten wurde die Compule aus der Heizeinheit entfernt und der Temperaturverlauf weiter alle 10 Sekunden aufgezeichnet. In einem Temperaturbereich von 45 bis 50 °C erfolgte ein Farbumschlag von rot zurück nach violett.

### Beispiel 3

Aus 5 Gew.-% thermochromen Masterbatch und 95 Gew.-% Polyethylen wurden Kolben gefertigt, die in ihren Abmessungen den Kolben von Grandio SO entsprachen. Aus einer Compule Grandio SO (VOCO GmbH) wurde der Kolben entfernt und durch einen thermochromen Kolben ersetzt. Die Compule wurde in der auf 68 °C vorgeheizten Heizeinheit (Caps Warmer - VOCO GmbH) platziert. Alle 10 Sekunden wurde die Temperatur des Kolbens gemessen. Zusätzlich wurde die Farbe des Kolbens beobachtet. In einem Temperaturbereich von 45 bis 50 °C erfolgte ein Farbumschlag von violett nach rot. Nach drei Minuten wurde die Compule aus der Heizeinheit entfernt und der Temperaturverlauf weiter alle 10 Sekunden aufgezeichnet. In einem Temperaturbereich von 45 bis 50 °C erfolgte ein Farbumschlag von rot zurück nach violett.

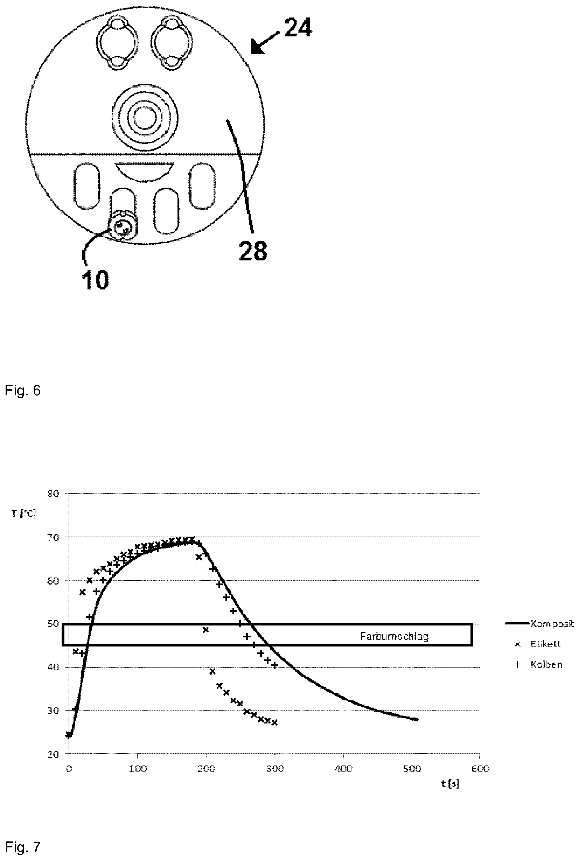

In Fig. 7 ist der Temperaturverlauf für das Dental- bzw. Kompositmaterial (20), den Kolben (18) und das Etikett dargestellt. Das Etikett auf der Oberfläche reagiert sowohl beim Aufwärmen als auch beim Abkühlen wesentlich schneller auf den Temperaturanstieg bzw. -abfall als der Kolben (18) und das Dentalmaterial (20), die sich im Innern der Compule befinden.

## Patentansprüche

1. Vorrichtung (10) zur Aufnahme und Applikation von Dentalmaterial (20), mit
einem Hohlraum (12) zur Aufnahme des Dentalmaterials (20) und
einem Kolben (18), der den Hohlraum an einer Seite verschließt und der entlang der Längsachse (L) in der Vorrichtung (10) beweglich ist, wobei der Kolben (18) zumindest teilweise aus einem thermochromen Material besteht
wobei die Vorrichtung (10) im Hohlraum (12) ein strahlungshärtbares Dentalmaterial (20) enthält, welches eine einkomponentige Kompositzusammensetzung ist, umfassend
(A)Monomere,
(B)Füllstoffe und
(C) Initiatoren.

2. Vorrichtung (10) nach Anspruch 1, wobei die einkomponentige Kompositzusammensetzung lichthärtbare ist und umfasst
(A) Monomere in einer Menge von 6 bis 35 Gew.-%, vorzugsweise 10 bis 35 Gew.-%, besonders bevorzugt 10 bis 25 Gew.-%, bezogen auf die Kompositzusammensetzung
(B) Füllstoffe in einer Menge von 65 bis 93 Gew.-%, vorzugsweise 65 bis 89 Gew.-%, besonders bevorzugt 75 bis 89 Gew.-%, bezogen auf die Kompositzusammensetzung,
(C) Initiatoren in einer Menge von 0,001 bis 3 Gew.-%, bezogen auf die Menge der Kompositzusammensetzung,
(D) weitere Additive in einer Menge von 0,001 bis 5 Gew.-%, bezogen auf die Menge der Kompositzusammensetzung.

3. Vorrichtung (10) nach einem der vorangehenden Ansprüche, wobei der Kolben (18) unterhalb der Schalttemperatur des thermochromen Materials eine erste Farbe aufweist und der oberhalb der Schalttemperatur des thermochromen Materials eine zweite Farbe aufweist, die von der ersten Farbe verschieden ist.

4. Vorrichtung (10) nach einem der vorangehenden Ansprüche, wobei der Kolben (18) in einem ersten Temperaturbereich von 10 bis 30 °C, bevorzugt in einem Temperaturbereich von 15 bis 25 °C, eine erste Farbe aufweist und in einem zweiten Temperaturbereich von 40 bis 80 °C, bevorzugt in einem Temperaturbereich von 50 bis 70 °C, eine zweite Farbe aufweist, die von der ersten Farbe verschieden ist.

5. Vorrichtung (10) nach einem der vorangehenden Ansprüche, wobei der Kolben (18) in einem ersten Temperaturbereich von 10 bis 30 °C, bevorzugt in einem Temperaturbereich von 15 bis 25 °C, eine erste Farbe aufweist und in einem zweiten Temperaturbereich von 40 bis 80 °C, bevorzugt in einem Temperaturbereich von 50 bis 70 °C, eine zweite Farbe aufweist, die von der ersten Farbe verschieden ist, und in einem dritten Temperaturbereich von größer 80 °C eine dritte Farbe aufweist, die von der zweiten Farbe, bevorzugt von der ersten und der zweiten Farbe, verschieden ist.

6. Vorrichtung (10) nach einem der vorangehenden Ansprüche, wobei der Körper (14) über einen Temperaturbereich von 10 bis 80°C eine Farbe aufweist, die der zweiten Farbe des Kolbens (18) im zweiten Temperaturbereich von 40 bis 80 °C, bevorzugt in einem Temperaturbereich von 50 bis 70 °C, entspricht.

7. Vorrichtung (10) nach einem der vorangehenden Ansprüche, wobei der Kolben (18) entlang der Längsachse (L) einen Transmissionsgrad von weniger als 10%, vorzugsweise weniger als 5%, besonders bevorzugt von weniger als 1%, für Strahlung im Wellenlängenbereich von 250 bis 500 nm, vorzugsweise im Wellenlängenbereich von 450 bis 480 nm, aufweist.

8. Vorrichtung (10) nach einem der vorangehenden Ansprüche **dadurch gekennzeichnet, dass** sie eine wasserdampf- und/oder lichtundurchlässige Verpackung umfasst.

9. Vorrichtung (10) nach einem der vorangehenden Ansprüche, wobei der Kolben (18) aus einem Kunststoff und einem thermochromen Pigment besteht, wobei der Kunststoff ausgewählt ist aus der Gruppe bestehend aus PE, PP, PVC, PS, PTFE, TPE, LSR, Silikon, EPDM, NBR, FKM, vorzugsweise ausgewählt ist aus der Gruppe bestehend aus PE, PP und PVC.

10. Vorrichtung (10) nach einem der vorhergehenden Ansprüche wobei der Kolben (18) im 2-Komponenten-Spritzgussverfahren aus zwei verschiedenen Materialien (18', 18") hergestellt wird, von denen das der Außenseite zugewandte Material (18") thermochrome Eigenschaften hat.

11. Vorrichtung (10) nach einem der vorangehenden Ansprüche, ausgewählt aus der Gruppe bestehend aus Spritzen, Applikationskanülen, Kapseln und Compulen.

12. Verfahren zum Erwärmen von Dentalmaterialien umfassend folgende Schritte
a) Bereitstellen einer Vorrichtung (10), definiert wie in einem der Ansprüche 1 bis 11,
b) Bereitstellen einer Heizeinheit (24),
c) Platzieren der Vorrichtung (10), in der Heizeinheit (24),
d) Erwärmen der Vorrichtung (10) und des darin enthaltenen Dentalmaterials (20) von einer ersten Temperatur unterhalb der Schalttemperatur des thermochromen Kolbenmaterials auf eine zweite Temperatur oberhalb der Schalttemperatur des thermochromen Kolbenmaterials,
e) Visuelles Erkennen, dass eine Temperatur oberhalb der Schalttemperatur erreicht ist am Farbwechsel des thermochromen Kolbenmaterials.

13. Verfahren nach Anspruch 12, wobei in Schritt d) von einer ersten Temperatur im Bereich von 10 bis 30 °C, vorzugsweise im Bereich von 15 bis 25 °C, auf eine zweite Temperatur im Bereich von 40 bis 80 °C, vorzugsweise im Bereich von 50 bis 70 °C, erwärmt wird.

14. Verfahren nach einem der Ansprüche 12 bis 13, wobei in Schritt e) die Farbe des thermochromen Kolbenmaterials mit der Farbe einer, bevorzugt auf der Heizeinheit (24) angebrachten, Referenzmarkierung oder Referenzskala abgeglichen wird, die die Farbe in Bezug zur Kolbentemperatur setzt.

15. Kit umfassend
- eine Vorrichtung (10) wie in den Ansprüchen 1 bis 10 definiert, und
- eine Heizeinheit (24).

16. Kit nach Anspruch 15, wobei die Heizeinheit (24) ein Referenzetikett, eine Referenzmarkierung oder eine Referenzskala aufweist, die der Farbe des Kolbens (18) eine Temperatur zuordnet.

## Claims

1. A device (10) for accommodating and applying dental material (20), having a cavity (12) for accommodating the dental material (20) and a piston (18) which closes the cavity on one side and which is movable along the longitudinal axis (L) in the device (10), wherein the piston (18) consists at least partly of a thermochromic material, wherein the device (10) in the cavity (12) contains a radiation-curable dental material (20) which is a one-component composite composition comprising
(A) monomers,
(B) fillers and
(C) initiators.

2. The device as claimed in claim 1, wherein the one-component composite composition, is light-curable and comprises
(A) monomers in an amount of 6% to 35% by weight, preferably 10% to 35% by weight, more preferably 10% to 25% by weight, based on the composite composition,
(B) fillers in an amount of 65% to 93% by weight, preferably 65% to 89% by weight, more preferably 75% to 89% by weight, based on the composite composition,
(C) initiators in an amount of 0.001% to 3% by weight, based on the amount of the composite composition,
(D) further additives in an amount of 0.001% to 5% by weight, based on the amount of the composite composition.

3. The device (10) as claimed in any of the preceding claims, wherein the piston (18) has a first color below the switching temperature of the thermochromic material and a second color different than the first color, above the switching temperature of the thermochromic material.

4. The device (10) as claimed in either of the preceding claims, wherein the piston (18) has a first color within a first temperature range from 10 to 30°C, preferably within a temperature range from 15 to 25°C, and a second color different than the first color within a second temperature range from 40 to 80°C, preferably within a temperature range from 50 to 70°C.

5. The device (10) as claimed in any of the preceding claims, wherein the piston (18) has a first color within a first temperature range from 10 to 30°C, preferably within a temperature range from 15 to 25°C, and a second color different than the first color within a second temperature range from 40 to 80°C, preferably within a temperature range from 50 to 70°C, and a third color different than the second color, preferably than the first and second colors, within a third temperature range of greater than 80°C.

6. The device (10) as claimed in any of the preceding claims, wherein the body (14), over a temperature range from 10 to 80°C, has a color corresponding to the second color of the piston (18) within the second temperature range from 40 to 80°C, preferably within a temperature range from 50 to 70°C.

7. The device (10) as claimed in any of the preceding claims, wherein the piston (18), along the longitudinal axis (L), has a transmittance of less than 10%, preferably less than 5%, more preferably less than 1%, for radiation in the wavelength range from 250 to 500 nm, preferably in the wavelength range from 450 to 480 nm.

8. The device (10) as claimed in any of the preceding claims, which comprises a water vapor-impermeable and/or opaque packing.

9. The device (10) as claimed in any of the preceding claims, wherein the piston (18) consists of a plastic and a thermochromic pigment, wherein the plastic is selected from the group consisting of PE, PP, PVC, PS, PTFE, TPE, LSR, silicone, EPDM, NBR, FKM, preferably selected from the group consisting of PE, PP and PVC.

10. The device (10) as claimed in any of the preceding claims, wherein the piston (18) is produced by a 2-component injection molding process from two different materials (18', 18"), of which the material (18") facing outward has thermochromic properties.

11. The device (10) as claimed in any of the preceding claims, selected from the group consisting of syringes, application cannulas, capsules and compules.

12. A method of heating dental materials, comprising the following steps:
a) providing a device (10) as defined in any of claims 1 to 11,
b) providing a heating unit (24),
c) placing the device (10) in the heating unit (24),
d) heating the device (10) and the dental material (20) present therein from a first temperature below the switching temperature of the thermochromic piston material to a second temperature above the switching temperature of the thermochromic piston material,
e) visually recognizing that a temperature above the switching temperature has been attained by the color change in the thermochromic piston material.

13. The method as claimed in claim 12, wherein heating is effected in step d) from a first temperature in the range from 10 to 30°C, preferably in the range from 15 to 25°C, to a second temperature in the range from 40 to 80°C, preferably in the range from 50 to 70°C.

14. The method as claimed in either of claims 12 and 13, wherein, in step e), the color of the thermochromic piston material is compared with the color of a reference marker or reference scale, preferably mounted on the heating unit (24), that relates the color to the piston temperature.

15. A kit comprising
- a device (10) as defined in claims 1 to 10, and
- a heating unit (24).

16. The kit as claimed in claim 15, wherein the heating unit (24) has a reference label, reference marker or reference scale that assigns a temperature to the color of the piston (18).

## Revendications

1. Dispositif (10) pour la réception et l'application de matériau dentaire (20), muni d'une cavité (12) pour la réception du matériau dentaire (20) et d'un piston (18), qui ferme la cavité sur un côté et qui est mobile le long de l'axe longitudinal (L) dans le dispositif (10), le piston (18) étant au moins partiellement constitué d'un matériau thermochromique,
le dispositif (10) contenant dans la cavité (12) un matériau dentaire durcissable par rayonnement (20), qui est une composition composite monocomposante, comprenant
(A) des monomères,
(B) des charges et
(C) des initiateurs.

2. Dispositif (10) selon la revendication 1, dans lequel la composition composite monocomposante est durcissable à la lumière et comprend
(A) des monomères en une quantité de 6 à 35 % en poids, de préférence 10 à 35 % en poids, de manière particulièrement préférée 10 à 25 % en poids, par rapport à la composition composite,
(B) des charges en une quantité de 65 à 93 % en poids, de préférence 65 à 89 % en poids, de manière particulièrement préférée 75 à 89 % en poids, par rapport à la composition composite,
(C) des initiateurs en une quantité de 0, 001 à 3 % en poids, par rapport à la quantité de la composition composite,
(D) des additifs supplémentaires en une quantité de 0,001 à 5 % en poids, par rapport à la quantité de la composition composite.

3. Dispositif (10) selon l'une quelconque des revendications précédentes, dans lequel le piston (18) présente une première couleur en dessous de la température de commutation du matériau thermochromique et présente une deuxième couleur, qui est différente de la première couleur, au-dessus de la température de commutation du matériau thermochromique.

4. Dispositif (10) selon l'une quelconque des revendications précédentes, dans lequel le piston (18) présente une première couleur dans une première plage de température allant de 10 à 30 °C, de préférence dans une plage de température allant de 15 à 25 °C, et présente une deuxième couleur, qui est différente de la première couleur, dans une deuxième plage de température allant de 40 à 80 °C, de préférence dans une plage de température allant de 50 à 70 °C.

5. Dispositif (10) selon l'une quelconque des revendications précédentes, dans lequel le piston (18) présente une première couleur dans une première plage de température allant de 10 à 30 °C, de préférence dans une plage de température allant de 15 à 25 °C, et présente une deuxième couleur, qui est différente de la première couleur, dans une deuxième plage de température allant de 40 à 80 °C, de préférence dans une plage de température allant de 50 à 70 °C, et présente une troisième couleur, qui est différente de la deuxième couleur, de préférence de la première et de la deuxième couleur, dans une troisième plage de température de plus de 80 °C.

6. Dispositif (10) selon l'une quelconque des revendications précédentes, dans lequel le corps (14) présente sur une plage de température allant de 10 à 80 °C une couleur qui correspond à la deuxième couleur du piston (18) dans la deuxième plage de température allant de 40 à 80 °C, de préférence dans une plage de température allant de 50 à 70 °C.

7. Dispositif (10) selon l'une quelconque des revendications précédentes, dans lequel le piston (18) présente le long de l'axe longitudinal (L) un degré de transmission de moins de 10 %, de préférence de moins de 5 %, de manière particulièrement préférée de moins de 1 %, pour le rayonnement dans la plage de longueur d'onde allant de 250 à 500 nm, de préférence dans la plage de longueur d'onde allant de 450 à 480 nm.

8. Dispositif (10) selon l'une quelconque des revendications précédentes, **caractérisé en ce qu'**il comprend un emballage imperméable à la vapeur d'eau et/ou à la lumière.

9. Dispositif (10) selon l'une quelconque des revendications précédentes, dans lequel le piston (18) est constitué d'une matière plastique et d'un pigment thermochromique, dans lequel la matière plastique est choisie dans le groupe constitué par le PE, le PP, le PVC, le PS, le PTFE, le TPE, le LSR, la silicone, l'EPDM, le NBR, le FKM, de préférence est choisie dans le groupe constitué par le PE, le PP et le PVC.

10. Dispositif (10) selon l'une quelconque des revendications précédentes, dans lequel le piston (18) est fabriqué dans un procédé de moulage par injection à 2 composants à partir de deux matériaux différents (18', 18ʺ), parmi lesquels le matériau (18ʺ) tourné vers le côté extérieur a des propriétés thermochromiques.

11. Dispositif (10) selon l'une quelconque des revendications précédentes, choisi dans le groupe constitué par les seringues, les canules d'application, les capsules et les compules.

12. Procédé de réchauffement de matériaux dentaires, comprenant les étapes suivantes :
a) la fourniture d'un dispositif (10), tel que défini dans l'une quelconque des revendications 1 à 11,
b) la fourniture d'une unité de chauffage (24),
c) la mise en place du dispositif (10) dans l'unité de chauffage (24),
d) le réchauffement du dispositif (10) et du matériau dentaire (20) contenu dans celui-ci d'une première température inférieure à la température de commutation du matériau de piston thermochromique à une deuxième température supérieure à la température de commutation du matériau de piston thermochromique,
e) l'identification visuelle qu'une température supérieure à la température de commutation est atteinte lors du changement de couleur du matériau de piston thermochromique.

13. Procédé selon la revendication 12, dans lequel, dans l'étape d), le réchauffement est effectué d'une première température dans la plage allant de 10 à 30 °C, de préférence dans la plage allant de 15 à 25 °C, à une deuxième température dans la plage allant de 40 à 80 °C, de préférence dans la plage allant de 50 à 70 °C.

14. Procédé selon l'une quelconque des revendications 12 à 13, dans lequel, dans l'étape e), la couleur du matériau de piston thermochromique est comparée à la couleur d'un marquage de référence ou d'une échelle de référence, de préférence disposé sur l'unité de chauffage (24), qui indique la couleur en fonction de la température du piston.

15. Kit comprenant
- un dispositif (10) tel que défini dans les revendications 1 à 10,
et
- une unité de chauffage (24).

16. Kit selon la revendication 15, dans lequel l'unité de chauffage (24) comprend une étiquette de référence, un marquage de référence ou une échelle de référence, qui attribue une température à la couleur du piston (18) .
